# EUROPÄISCHE PATENTSCHRIFT

(11) **EP 2 196 237 B1**
(45) Veröffentlichungstag und Bekanntmachung des Hinweises auf die Patenterteilung: **29.04.2015**
(21) Anmeldenummer: 09015126.7
(22) Anmeldetag: 07.12.2009
(51) Int. Cl.: A61N 1/04, A61B 5/0408, A61B 18/16

(54) **Bioelektrode**
Bioelectrode
Bioélectrode

(30) Priorität: 12.12.2008 AT 19342008
(43) Veröffentlichungstag der Anmeldung: 16.06.2010
(73) Patentinhaber: Leonh. Lang, 6020 Innsbruck (AT)
(72) Erfinder: Wilfinger, Markus, 6063 Rum (AT)
(74) Vertreter: Hofinger, Stephan

(56) Entgegenhaltungen:
- EP-A1- 0 097 436
- EP-A1- 1 905 479
- WO-A1-98/41278
- US-A1- 2005 015 134
- ANONYMOUS: "Typical Properties for 3M Electrically Conductive Adhesive Films" 3M TECHNICAL BULLETIN, [Online] Februar 2000 (2000-02), Seiten 1-4, XP002572097 Gefunden im Internet: URL:http://multimedia.3m.com/mws/mediawebs erver?66666UuZjcFSLXTtmXfVMxT2EVuQEcuZgVs6 EVs6E666666--> [gefunden am 2010-03-09]

## Beschreibung

Die Erfindung betrifft eine Bioelektrode, mit einer hautseitigen, elektrisch leitenden Klebeschicht, einem flexiblen elektrischen Anschlusskabel, das in einem elektrisch isolierenden Kabelmantel zumindest einen elektrischen Leiter, vorzugsweise in Form einer aus mehreren Einzeldrähten bzw. leitenden Einzelfasern bestehenden Litze, umfasst, und zwei thermoplastischen Schichten die elektrisch leitend sind und mit dem elektrischen Leiter des Anschlusskabels elektrisch in Verbindung stehen, wobei das elektrodenseitige Ende des Anschlusskabels zwischen den zwei thermoplastischen Schichten angeordnet ist.

Bioelektroden kommen in vielfacher Weise zum Einsatz. Es wird entweder wie bei Defibrillationselektroden und Stimulationselektroden Strom dem menschlichen oder tierischen Körper zugeführt oder Strom vom Körper abgeführt (beispielsweise Neutralelektroden oder Messelektroden).

Aus der US 2005/0015134 A1 geht hervor, die Enden eines Anschlusskabels zwischen zwei elektrisch leitenden Klebebändern anzuordnen, wobei diese beiden Klebebänder nur auf einfache Art und Weise miteinander verklebt sind und somit einen relativ schlechten mechanischen Halt und schlechte elektrische Verbindung zu den Enden des Anschlusskabels ergeben.

Aus der XP-002572097 des 3M Technical Bulletin gehen typische Eigenschaften für elektrisch leitende Klebefilme hervor, wobei beispielsweise unter der Nr. 9713 ein elektrisch leitender Klebefilm gezeigt ist, der bei Raumtemperatur und geringem Druck miteinander verbunden werden kann. Nachteilig dabei ist, dass diese Verbindung lösbar ist und dass nur der Klebefilm des Klebebands elektrisch leitend ist.

Aufgabe der Erfindung ist es, eine Bioelektrode zu schaffen, bei der das elektrische Anschlusskabel einen guten mechanischen Halt in der Elektrode hat und außerdem ein guter elektrischer Kontakt zu jenen Schichten der Elektrode gewährleistet ist, die letztlich den Strom der Haut zuführen oder von dieser abnehmen.

Erfindungsgemäß wird dies dadurch erreicht, dass die zwei thermoplastischen Schichten zumindest bereichsweise miteinander verschweißt sind, wobei die beiden thermoplastischen Schichten unter Einschluss des elektrodenseitigen Anschlusskabels thermisch oder mittels Ultraschall miteinander verschweißt sind.

Die erfindungsgemäße Anbindung des elektrodenseitigen Anschlussendes zwischen zwei thermisch oder ultraschall-verschweißten thermoplastischen Schichten erlaubt es, einerseits einen guten mechanischen Halt zu erzielen, und gleichzeitig eine hervorragende elektrische Verbindung mit den beiden thermoplastischen Schichten herzustellen, die elektrisch leitend ausgebildet sind.

Die elektrisch leitenden, thermoplastischen Schichten erlauben es, den über das elektrische Anschlusskabel zugeführten Strom über eine größere Fläche gleichmäßig zu verteilen bzw. von einer größeren Fläche abzunehmen. Es ist aber auch möglich, in der elektrisch leitenden thermoplastischen Schicht, die auch aus mehreren Unterschichten bestehen kann, ein spezielles Widerstandsprofil einzubauen, beispielsweise derart, dass der Flächenwiderstand vom mittleren Anschlusspunkt des elektrodenseitigen Kabelendes zum Rand der elektrisch leitenden thermoplastischen Schicht je nach Bedarf abnimmt oder zunimmt. Jedenfalls ist eine gezielte flächige Stromverteilung möglich.

Weitere Vorteile und Einzelheiten der Erfindung werden anhand der nachfolgenden Figurenbeschreibung näher erläutert.

Die Fig. 1a zeigt ein erstes Ausführungsbeispiel der Erfindung in einer schematischen Explosionsdarstellung. Fig. 1b zeigt die Elektrode der Fig. 1a in einer schematischen Querschnittsdarstellung. Fig. 1 c zeigt einen Kabelquerschnitt.

Die Fig. 2a zeigt ein zweites Ausführungsbeispiel der Erfindung in einer schematischen Explosionsdarstellung. Fig. 2b zeigt die Elektrode der Fig. 2a in einer schematischen Querschnittsdarstellung.

Die Fig. 3a zeigt ein drittes Ausführungsbeispiel der Erfindung in einer schematischen Explosionsdarstellung. Fig. 3b zeigt die Elektrode der Fig. 3a in einer schematischen Querschnittsdarstellung.

Die Fig. 4a zeigt ein viertes Ausführungsbeispiel der Erfindung in einer schematischen Explosionsdarstellung. Fig. 4b zeigt die Elektrode der Fig. 4a in einer schematischen Querschnittsdarstellung.

Die Fig. 5a zeigt ein fünftes Ausführungsbeispiel der Erfindung in einer schematischen Explosionsdarstellung. Fig. 5b zeigt die Elektrode der Fig. 5a in einer schematischen Querschnittsdarstellung.

Die Fig. 6a zeigt ein sechstes Ausführungsbeispiel der Erfindung in einer schematischen Explosionsdarstellung. Fig. 6b zeigt die Elektrode der Fig. 6a in einer schematischen Querschnittsdarstellung.

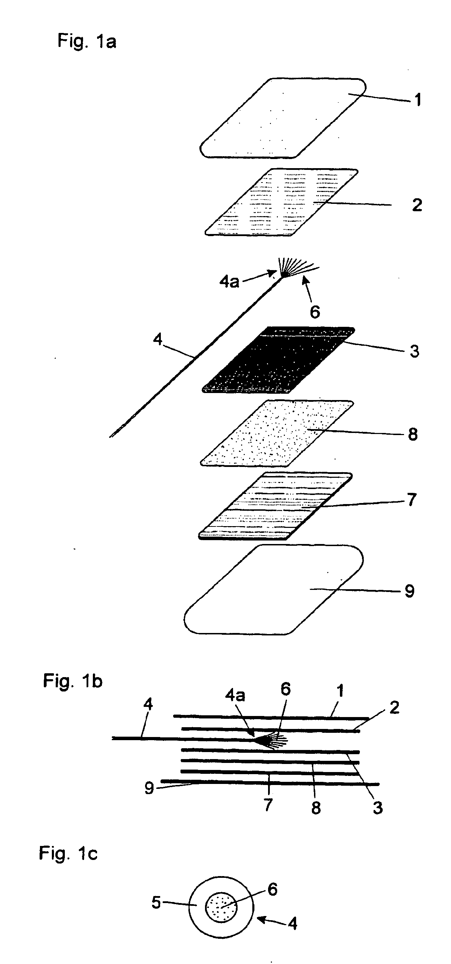

Die Fig. 1a und 1b zeigen ein erstes Ausführungsbeispiel einer erfindungsgemäßen Elektrode, insbesondere Defibrillationselektrode.

Die Elektrode der Fig. 1a und 1b weist unterhalb eines Trägermateriales 1 (z.B.: Schaummaterial bestehend aus Polyethylen oder ähnlichem, Folie bestehend aus Polyethylenterephthalat oder ähnlichem) zwei thermoplastische Schichten 2, 3 auf, von denen zumindest die untere (also hautseitig liegende) Schicht 3 elektrisch leitend ausgebildet ist. Die thermoplastischen Schichten (2, 3) können z.B.: aus Polyvinylchlorid, Acrylbutadienstyrol, Polyurethan, Polyethylen oder ähnlichem bestehen. Die elektrische Leitfähigkeit der thermoplastischen Schicht 3 kann beispielsweise durch metallische Einschlüsse und/oder Einschlüsse auf Kohlenstoffbasis (Ruß, Graphit) erzielt werden. Durch eine entsprechende Verteilung dieser Einschlüsse oder eine Mehrschichtigkeit der Schicht 3 kann man den elektrischen Widerstand über die Elektrode variieren.

Erfindungsgemäß ist nun das elektrodenseitige freie Ende 4a des Anschlusskabels 4 zwischen den beiden thermoplastischen Schichten 2, 3 angeordnet, die miteinander zumindest bereichsweise thermisch oder ultraschall-verschweißt sind. Es wird darauf hingewiesen, dass die Querschnittsdarstellung gemäß der Fig. 1b nur die Schichtfolge anzeigt, die einzelnen Schichten der Elektrode aber natürlich direkt aneinander liegen und miteinander verbunden sind. Insofern liegt auch der schematisch dargestellte Fächer der Einzeldrähte/Einzelfasern der Litze horizontal flach, wie in Fig. 1a gezeigt. Die Schicht 2 ist mit der Schicht 3 unter Einschluss des elektrodenseitigen Endes 4a des Anschlusskabels 4 zumindest bereichsweise innig miteinander verschweißt. Damit hat das Kabelende 4a einen guten mechanischen Halt in der Elektrode und außerdem einen hervorragenden elektrischen Kontakt zur leitenden thermoplastischen Schicht 3.

Das elektrische Anschlusskabel 4 umfasst - wie in Fig. 1c im Querschnitt dargestellt - einen elektrisch isolierenden Kabelmantel 5, in dem sich zumindest ein elektrischer Leiter 6 befindet. Bevorzugt ist dieser elektrische Leiter 6 eine Litze, die aus mehreren Einzeldrähten/Einzelfasem besteht.

Verwendbare Materialien des isolierenden Kabelmantels sind Polyethylen, Polypropylen oder Polyvinylchlordid oder ähnliches. Die Leitadern können Karbonfaserstränge sein, die aus mehreren 1000 bis mehreren 10.000 Einzelfasern bestehen, welche metallisiert sein können. Als Leitader können auch Metalllitzen alleine oder Metalllitzen kombiniert mit Karbonfasern verwendet werden. Eine derartige Kabelausbildung erlaubt es, das Kabelende beispielsweise auf einer Länge von 0,5 cm bis 2 cm abzuisolieren, und dann die Einzeldrähte der Litze aufzufächern, wie dies in den Fig. 1 a und 1b schematisch dargestellt ist. Durch eine solche Auffächerung lässt sich ein noch besserer mechanischer und elektrischer Kontakt des elektrodenseitigen Kabelendes 4a mit der elektrisch leitenden thermoplastischen Schicht 3 herstellen. Die beiden thermoplastischen Schichten 2, 3 werden unter Einschluss des elektrodenseitigen freien Endes 4a des Anschlusskabels 4 zumindest bereichsweise miteinander thermisch verschweißt.

Bei einer Schichtstärke in der Größenordnung von 50 bis 150 Mikrometer der thermoplastischen Schichten 2, 3 kann man diese Verschweißung bei einer Temperatur zwischen 150° C und 200 °C unter Aufbringung eines Druckes von ca. 1 N/cm² bis 5 N/cm² vornehmen, wobei die Verschweißdauer günstigerweise zwischen 5 sec und 20 sec liegt.

Alternativ kann erfindungsgemäß auch ein Ultraschallsehweißverfahren zum Einsatz kommen. Hier ist eine Arbeitsfrequenz in der Größenordnung von beispielsweise 20 KHz geeignet. Der Energieeintrag liegt günstigerweise zwischen 200 Ws und 600 Ws. Der Anpressdruck liegt günstigerweise in der Größenordnung zwischen 50 N/cm² und 100 N/cm². Die Verschweißzeiten mittels Ultraschallverschweißung sind recht kurz und liegen günstigerweise unter einer Sekunde.

Bei dem in Fig. 1a und 1b dargestellten Ausführungsbeispiel ist nur die untere der beiden thermoplastischen Schichten, nämlich die Schicht 3, elektrisch leitend ausgebildet. Es kann jedoch auch die obere thermoplastische Schicht 2 zumindest teilweise elektrisch leitend ausgebildet sein. Diese obere thermoplastische Schicht 2 kann auch die oberste Trägerschicht 1 ersetzen, sodass diese entfallen kann. Die thermoplastische Schicht 2 kann beispielsweise aus Acrylbutadienstyrol, Polyethylen, Polyurethan, Polyvinylchlorid oder ähnlichem bestehen. Wird die thermoplastische Schicht 2 zumindest teilweise elektrisch leitfähig ausgebildet, muss diese mit elektrisch leitenden Füllstoffen, insbesondere Pigmenten oder Fasern gefüllt sein, oder wie Schicht 3 aufgebaut sein. Wird die thermoplastische Schicht 2 als Trägerschicht 1 ausgebildet, darf die Schicht 2 nicht elektrisch ausgebildet sein.

Hautseitig weist die Bioelektrode nach den Fig. 1a bis 1b eine leitfähige Klebeschicht 7, vorzugsweise in Form eines leitfähigen Gels, auf. Die leitfähige Klebeschicht, welche biokompatibel sein muss, kann sowohl ein klebendes Hydrogel, als auch ein leitfähiger Kleber sein.

Zwischen der hautseitigen leitfähigen Klebeschicht 7 und der elektrisch leitenden thermoplastischen Schicht 3 kann eine Metallschicht oder eine Metall-/Metallchloridschicht angeordnet sein, wobei das Metall vorzugsweise Silber ist. Diese Schicht trägt das Bezugszeichen 8.

Unterhalb der leitfähigen Klebeschicht 7 ist ein abziehbares Abdeckmaterial 9 angeordnet, das die leitfähige Klebeschicht beim Transport und der Lagerung schützt, und das vor Benutzung abgezogen wird. Dieses Abdeckmaterial kann aus Kunststoffen wie Polyethylenterephthalat, Polystyrol, Polypropylen oder ähnlichem bestehen, welche auch silikonisiert sein können.

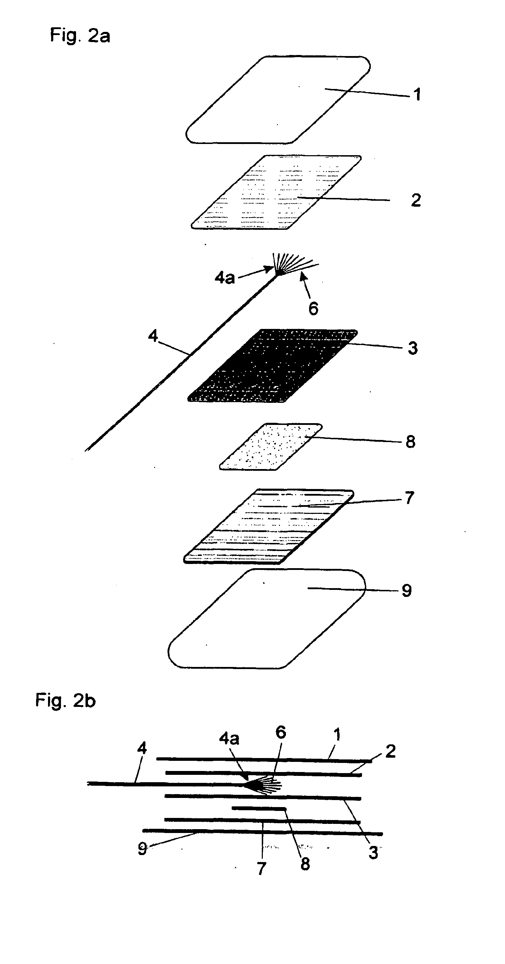

Das zweite Ausführungsbeispiel gemäß den Fig. 2a und 2b unterscheidet sich vom ersten Ausführungsbeispiel gemäß den Fig. 1a und 1b im Wesentlichen nur dadurch, dass die Zwischenschicht 8 (also die Metallschicht oder die Metall-/Metalichloridschicht) flächenmäßig kleiner ausgebildet ist als die darüberliegenden thermoplastischen Schichten 2, 3.

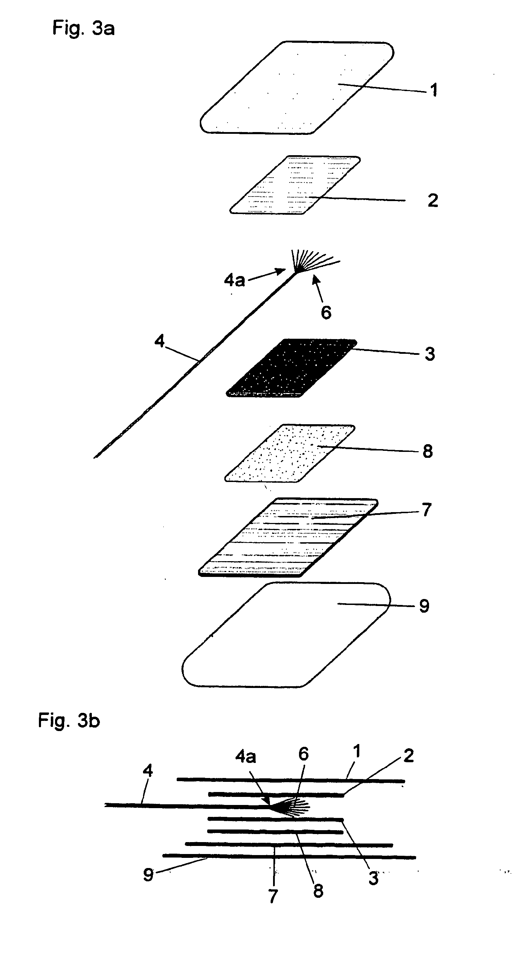

Beim dritten Ausführungsbeispiel gemäß den Fig. 3a und 3b sind auch die thermoplastischen Schichten 2, 3 kleiner ausgebildet als die hautseitige leitfähige Klebeschicht 7. Das Kabelende 4a kann dennoch mechanisch gut und elektrisch hervorragend kontaktiert zwischen den beiden Schichten 2, 3 gehalten sein. Während die untere der beiden thermoplastischen Schichten, nämlich die Schicht 3, eine elektrische Stromverteilung über die Fläche vornimmt, kann die leitfähige Klebeschicht 7 diese Stromverteilung noch auf einen größeren Flächenbereich erweitern.

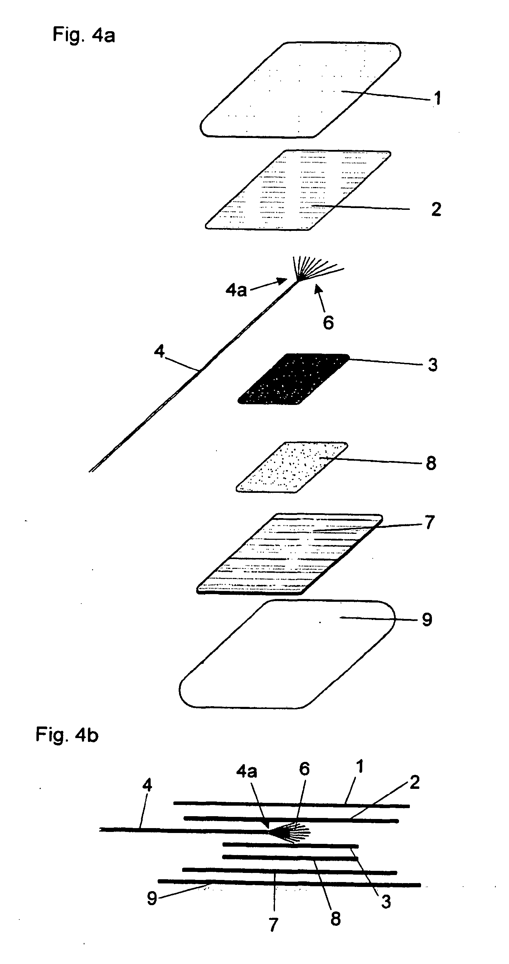

Beim Ausführungsbeispiel gemäß Fig. 4a und 4b sind die beiden thermoplastischen Schichten im Gegensatz zu den vorher genannten Ausführungsbeispielen nicht gleich groß, sondern verschieden groß. Die obere, nicht leitende thermoplastische Schicht 2 entspricht im Wesentlichen der sonstigen Größe der Elektrode, während die untere, elektrisch leitfähige Schicht 3 kleiner ausgebildet ist.

Die leitende Metallschicht ist bevorzugt ein Lack. Dieser ist zumindest mit Metallpigmenten versetzt. Weiters können Metallsalze eingearbeitet sein, welche mit ihren jeweiligen Metallen ein konstantes Eigenpotential bilden z.B.: Ag/AgCl. Die Metallschicht kann auch durch Bedampfung aufgebracht werden.

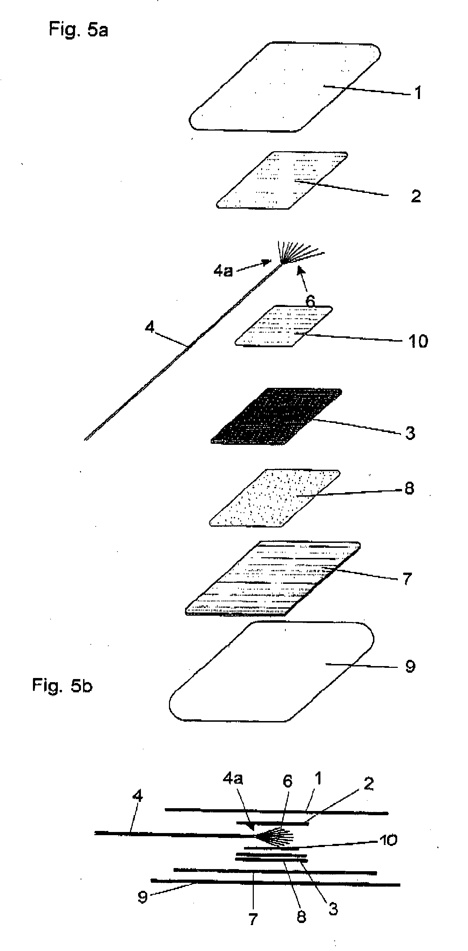

Beim fünften Ausführungsbeispiel gemäß den Fig. 5a und 5b liegt zwischen den beiden thermoplastischen, miteinander verschweißten Schichten 2, 3 noch eine zusätzliche elektrisch leitende, vorzugsweise metallische Schicht 10. Die Fläche der metallischen Schicht 10 ist dabei kleiner als die Fläche der beiden thermoplastischen Schichten 2, 3. Dies erlaubt es, die beiden thermoplastischen Schichten im überstehenden Bereich umfangseitig um die metallische Schicht 10 herum miteinander zu verschweißen. Dabei kann auch der Kabelmantel 5 des Anschlusskabels 4 bei geeignetem Material mit den beiden thermoplastischen Schichten 2, 3 verschweißt werden. Dieses Merkmal kann übrigens auch bei den anderen Ausführungsbeispielen eingesetzt werden.

Es kann auch ein Folienverbund als Schicht 10 verwendet werden (z.B.: Metall/Polyethylenterphthalat/Siegellack) wobei die isolierende Seite hautseitig ausgerichtet ist. In diesem Fall kann durch Wahl einer speziellen Geometrie der Schicht 10 (z.B.: sternförmig) der Strom optimal von der Schicht 10, über die thermoplastische Schicht 2 zur thermoplastischen Schicht 3, und den weiteren elektrischen leitenden Schichten, zur Haut verteilt werden.

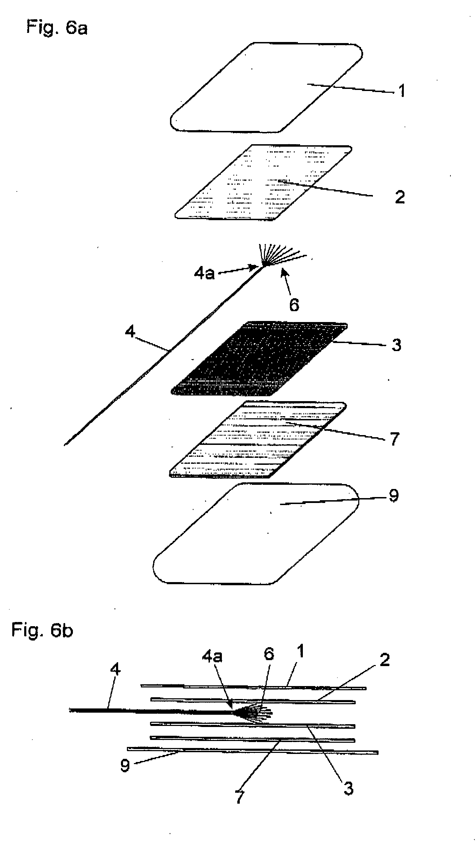

Beim sechsten Ausführungsbeispiel gemäß den Fig. 6a und 6b fehlt die Zwischenschicht 8 (Metallschicht bzw. Metall-/Metallchloridschicht). Damit grenzt die hautseitige leitfähige Klebeschicht 7 direkt an die untere, elektrisch leitende thermoplastische Schicht 3 an.

Die Erfindung ist selbstverständlich nicht auf die dargestellten Ausführungsbeispiele beschränkt. Beispielsweise eignet sich die Erfindung nicht nur für Defibrillationselektroden und Elektroden, die Strom der Haut zuführen (z.B.: Stimulationselektroden), sondern grundsätzlich auch für Elektroden, die Strom von der Haut abnehmen (beispielsweise Neutralelektroden, Messelektroden). Auch der Schichtaufbau und die Größenverhältnisse können von den gezeigten Ausführungsbeispielen abweichen. Wesentlich ist, dass das elektrodenseitige Ende des Anschlusskabels zwischen zwei thermoplastisch miteinander thermisch oder ultraschall-verschweißten Schichten angeordnet ist, sodass ein guter mechanischer Halt und eine gute elektrische Verbindung gewährleistet sind.

## Patentansprüche

1. Bioelektrode, mit
- einer hautseitigen, elektrisch leitenden Klebeschicht (7),
- einem flexiblen elektrischen Anschlusskabel (4), das in einem elektrisch isolierenden Kabelmantel (5) zumindest einen elektrischen Leiter (6), umfasst, und
- zwei thermoplastischen Schichten (2, 3) die elektrisch leitend sind und mit dem elektrischen Leiter (6) des Anschlusskabels (4) elektrisch in Verbindung stehen, wobei das elektrodenseitige Ende (4a) des Anschlusskabels (4) zwischen den zwei thermoplastischen Schichten (2, 3) angeordnet ist,
**dadurch gekennzeichnet, dass** die zwei thermoplastischen Schichten (2, 3) unter Einschluss des Endes (4a) des Anschlusskabels (4) zumindest bereichsweise thermisch miteinander verschweißt sind oder unter Einschluss des Endes (4a) des Anschlusskabels (4) zumindest bereichsweise ultraschall-verschweißt sind.

2. Bioelektrode nach Anspruch 1, **dadurch gekennzeichnet, dass** beide thermoplastischen Schichten (2, 3) durch metallische Einschlüsse und/oder Einschlüsse auf Kohlenstoffbasis elektrisch leitend sind.

3. Bioelektrode nach Anspruch 1 oder 2, **dadurch gekennzeichnet, dass** das Ende (4a) des Anschlusskabels (4) abisoliert ist, sodass der Leiter (6) auf einer zwischen 0,5 cm und 2 cm liegenden Länge freiliegt und in diesem abisolierten Bereich einen elektrischen Kontakt zur elektrisch leitenden thermoplastischen Schicht (3) aufweist.

4. Bioelektrode nach Anspruch 3, **dadurch gekennzeichnet, dass** die Einzeldrähte des Reiters (6) des elektrischen Anschlusskabels (4) im Bereich des abisolierten Endes aufgefächert sind und zwischen den beiden thermoplastischen Schichten (2, 3) liegen.

5. Bioelektrode nach einem der Ansprüche 1 bis 4, **dadurch gekennzeichnet, dass** die beiden thermoplastischen Schichten (2, 3) dieselbe Größe aufweisen.

6. Bioelektrode nach einem der Ansprüche 1 bis 5, **dadurch gekennzeichnet, dass** zwischen den beiden thermoplastischen Schichten (2, 3) eine elektrisch leitende, metallische Schicht (10) eingelegt ist, die mit dem elektrischen Leiter (6) des Anschlusskabels (4) elektrisch in Kontakt steht, wobei die beiden thermoplastischen Schichten größer ausgebildet sind als die metallische Schicht (10) und in dem über die metallische Schicht (10) vorstehenden Bereich miteinander verschweißt sind.

7. Bioelektrode nach Anspruch 6, **dadurch gekennzeichnet, dass** die thermoplastischen Schichten allseitig über die metallische Schicht (10) überstehen und im gesamten Umfangsbereich miteinander verschweißt sind.

8. Bioelektrode nach einem der Ansprüche 1 bis 7, **dadurch gekennzeichnet, dass** die elektrisch leitende hautseitige Klebeschicht (7) aus einem leitfähigen Hydrogel oder elektrisch leitendem Kleber besteht.

9. Bioelektrode nach einem der Ansprüche 1 bis 8, **dadurch gekennzeichnet, dass** zwischen der hautseitigen, elektrisch leitenden Klebeschicht (7) und der hautseitigen (3) der beiden thermoplastischen (2, 3) Schichten eine Metallschicht (8) oder eine Metall-/Metallchloridschicht (8) angeordnet ist.

10. Bioelektrode nach Anspruch 9, **dadurch gekennzeichnet, dass** das Metall Silber ist.

11. Bioelektrode nach einem der Ansprüche 1 bis 10. **dadurch gekennzeichnet, dass** hautseitig der leitfähigen Klebeschicht (7) ein abziehbares Abdeckmaterial (9) angeordnet ist.

12. Bioelektrode nach einem der Ansprüche 1 bis 11, **dadurch gekennzeichnet, dass** auf der der Haut abgewandten Oberseite der Elektrode ein elektrisch nicht leitendes Trägermaterial (1) aus Kunststoff angeordnet ist.

## Claims

1. Bioelectrode comprising
- a skin-side, electrically conducting adhesive layer (7),
- a flexible electrical connecting cable (4) which in an electrically insulating cable sheath (5) includes at least one electrical conductor (6), and
- two thermoplastic layers (2, 3) which are electrically conducting and are electrically connected to the electrical conductor (6) of the connecting cable (4), wherein the electrode-side end (4a) of the connecting cable (4) is arranged between the two thermoplastic layers (2, 3),
**characterized in that** the two thermoplastic layers (2, 3) are thermally welded together at least region-wise with including there between the end (4a) of the connecting cable (4) or that two thermoplastic layers (2, 3) are welded together at least region-wise by ultrasound with including there between the end (4a) of the connecting cable (4).

2. Bioelectrode according to claim 1, **characterized in that** both thermoplastic layers (2, 3) are electrically conducting by metallic inclusions or carbon-based inclusions or by metallic inclusions and carbon-based inclusions.

3. Bioelectrode according to claim 1 or 2, **characterized in that** the end (4a) of the connecting cable (4) is stripped so that the conductor (6) is exposed over a length preferably between 0.5 cm and 2 cm and **in that** stripped region has electrical contact with the electrically conducting thermoplastic layer (3).

4. Bioelectrode according to claim 3, **characterized in that** the individual wires of the electrical conductor (6) of the electrical connecting cable (4) are fanned open in the region of the stripped end and lie between the two thermoplastic layers (2, 3).

5. Bioelectrode according to one of claims 1 to 4, **characterized in that** the two thermoplastic layers (2, 3) are of substantially the same size.

6. Bioelectrode according to one of claims 1 to 5, **characterized in that** inserted between the two thermoplastic layers (2, 3) is an electrically conducting metallic layer (10) which is electrically in contact with the electrical conductor (6) of the connecting cable (4), wherein the two thermoplastic layers (2, 3) are larger than the metallic layer (10) and are welded together in the region projecting beyond the metallic layer (10).

7. Bioelectrode according to claim 6, **characterized in that** the thermoplastic layers (2, 3) project beyond the metallic layer (10) on all sides and are welded together in the entire peripheral region.

8. Bioelectrode according to one of claims 1 to 7, **characterized in that** the electrically conducting skin-side adhesive layer (7) comprises a conductive hydrogel or electrically conducting adhesive.

9. Bioelectrode according to one of claims 1 to 8, **characterized in that** a metal layer (8) or a metal/metal chloride layer (8) is arranged between the skin-side, electrically conducting adhesive layer (7) and the skin-side layer (3) of the two thermoplastic layers (2, 3).

10. Bioelectrode according to claim 9, **characterized in that** the metal is silver.

11. Bioelectrode according to one of claims 1 to 10, **characterized in that** a cover material (9) which can be pulled off is arranged on the skin-side of the conductive adhesive layer (7).

12. Bioelectrode according to one of claims 1 to 11, **characterized in that** an electrically non-conducting carrier material (1) made of plastic is arranged on the top side of the electrode, that is remote from the skin.

## Revendications

1. Bioélectrode, avec
- une couche adhésive (7) côté peau, électriquement conductrice,
- un câble de connexion (4) électrique flexible qui, dans une gaine de câble (5) électriquement isolante, comprend au moins un conducteur (6) électrique, et
- deux couches thermoplastiques (2, 3) qui sont électriquement conductrices et sont en liaison électrique avec le conducteur (6) électrique du câble de connexion (4), l'extrémité côté électrode (4a) du câble de connexion (4) étant disposée entre les deux couches thermoplastiques (2, 3),
**caractérisée en ce que** les deux couches thermoplastiques (2, 3) sont, au moins par régions, soudées l'une à l'autre thermiquement avec inclusion de l'extrémité (4a) du câble de connexion (4) ou sont, au moins par régions, soudées par ultrasons avec inclusion de l'extrémité (4a) du câble de connexion (4).

2. Bioélectrode selon la revendication 1, **caractérisée en ce que** les deux couches thermoplastiques (2, 3) sont électriquement conductrices par des inclusions métalliques et/ou par des inclusions à base de carbone.

3. Bioélectrode selon la revendication 1 ou 2, **caractérisée en ce que** l'extrémité (4a) du câble de connexion (4) est dénudée de telle sorte que le conducteur (6) est dégagé sur une longueur comprise entre 0,5 cm et 2 cm et présente dans cette zone dénudée un contact électrique avec la couche thermoplastique (3) électriquement conductrice.

4. Bioélectrode selon la revendication 3, **caractérisée en ce que** les fils individuels du conducteur (6) du câble de connexion (4) électrique sont séparés dans la zone de l'extrémité dénudée et sont situés entre les deux couches thermoplastiques (2, 3).

5. Bioélectrode selon une des revendications 1 à 4, **caractérisée en ce que** les deux couches thermoplastiques (2, 3) présentent la même grandeur.

6. Bioélectrode selon une des revendications 1 à 5, **caractérisée en ce que**, entre les deux couches thermoplastiques (2, 3), il est inséré une couche métallique (10) électriquement conductrice qui est en contact électrique avec le conducteur (6) électrique du câble de connexion (4), les deux couches thermoplastiques étant constituées de façon plus grande que la couche métallique (10) et étant soudées l'une à l'autre dans la zone dépassant au-dessus de la couche métallique (10).

7. Bioélectrode selon la revendication 6, **caractérisée en ce que** les couches thermoplastiques dépassent de tous côtés au-dessus de la couche métallique (10) et sont soudées l'une à l'autre dans la totalité de la zone circonférentielle.

8. Bioélectrode selon une des revendications 1 à 7, **caractérisée en ce que** la couche adhésive (7) côté peau, électriquement conductrice, se compose d'un hydrogel conducteur ou d'un adhésif électriquement conducteur.

9. Bioélectrode selon une des revendications 1 à 8, **caractérisée en ce que**, entre la couche adhésive (7), côté peau, électriquement conductrice et la couche côté peau parmi les deux couches thermoplastiques (2, 3), il est disposé une couche en métal (8) ou une couche en métal/chlorure métallique (8).

10. Bioélectrode selon la revendication 9, **caractérisée en ce que** le métal est de l'argent.

11. Bioélectrode selon une des revendications 1 à 10, **caractérisée en ce qu'**un matériau de recouvrement (9) pelable est disposé sur le côté peau de la couche adhésive (7) conductrice.

12. Bioélectrode selon une des revendications 1 à 11, **caractérisée en ce que**, sur le côté supérieur de l'électrode qui est éloigné de la peau, il est disposé un matériau support (1) électriquement non conducteur, en matière plastique.
